# EUROPEAN PATENT APPLICATION

(11) **EP 3 520 673 A1**
(43) Date of publication of application: **07.08.2019**
(21) Application number: 17858186.4
(22) Date of filing: 19.09.2017
(51) Int. Cl.: A61B 1/00, A61B 1/045

(54) **ENDOSCOPE SYSTEM AND OPERATION METHOD THEREFOR**

(30) Priority: 03.10.2016 JP 2016195345
(71) Applicant: FUJIFILM Corporation, Tokyo 106-8620 (JP)
(72) Inventor: AOYAMA, Tatsuya, Ashigarakami-gun Kanagawa 258-8538 (JP)
(74) Representative: Klunker IP Patentanwälte PartG mbB
(86) International application number: PCT/JP2017/033637
(87) International publication number: WO 2018/066347

(57) **Abstract**

An endoscope system which can reduce visual discomfort even in a case where a normal image and a special image are alternately displayed, and a method of operating the endoscope system are provided. In a case where an observation target including a first structure is illuminated with first mode illumination light, a first mode display image obtained by imaging the observation target is acquired. In a case where the observation target is illuminated with second mode illumination light, a second mode display image obtained by imaging the observation target is acquired. A corrected image is obtained by correcting the first mode display image or the second mode display image such that a difference in a color and/or brightness of the first structure between the first mode display image and the second mode display image before the correction becomes small.

## Description

### BACKGROUND OF THE INVENTION

### 1. Field of the Invention

The present invention relates to an endoscope system which displays a normal image of a natural color and a special image having high visibility of a vascular structure, and a method of operating the endoscope system.

### 2. Description of the Related Art

In a medical field, diagnosis using an endoscope system comprising a light source device, an endoscope, and a processor device has been performed widely. The endoscope system irradiates an observation target via the endoscope with illumination light emitted from the light source device, and the processor device generates an image of the observation target on the basis of an image signal obtained by imaging the observation target illuminated with the illumination light. By displaying the image on a monitor, a doctor can perform diagnosis while viewing the image on the monitor.

In recent years, in addition to a normal image in which an observation target is displayed in natural colors by using, as illumination light, broadband light such as white light with a wavelength range from a blue range to a red range, a special image in which the visibility of a specific structure such as a vascular structure is improved is also used for observation of the observation target by using, as illumination light, light with a specific wavelength and light of a plurality of colors in which the intensity of light of a specific color is made larger than the intensity of light of the other colors. Since it is necessary to use the normal image and the special image appropriately according to the purpose of diagnosis, a switch type mode switching unit is provided in an operating part of an endoscope such that a normal mode in which the normal image is displayed and a special mode in which the special image is displayed can appropriately be switched (refer to JP2008-043604A).

### SUMMARY OF THE INVENTION

In a case where display switching of a normal image and a special image is performed by a mode switching unit as in JP2008-043604A, since a user needs to operate the mode switching unit, a burden is placed on the user. Therefore, it is conceivable to display the normal image and the special image alternately on the monitor so that both the normal image and the special image can be observed in one mode. However, in a case where the normal image and the special image are alternately displayed as described above, since the normal image and the special image are different in color and brightness, sometimes, the user experiences visual discomfort such as eye flickering in a case where the display is switched from the normal image to the special image.

An object of the invention is to provide an endoscope system which can reduce visual discomfort such as eye flickering even in a case where the normal image and the special image are alternately displayed, and a method of operating the endoscope system.

An endoscope system according to the invention comprises: a light source that emits first mode illumination light and second mode illumination light; an image acquisition unit that acquires a first mode display image and a second mode display image, the first mode display image being obtained by imaging an observation target in a case where the observation target including a first structure is illuminated with the first mode illumination light, and the second mode display image being obtained by imaging the observation target in a case where the observation target is illuminated with the second mode illumination light; and an image correction unit that obtains a corrected image by correcting the first mode display image or the second mode display image, and performs correction such that a difference in a color and/or brightness of the first structure between the first mode display image and the second mode display image before the correction becomes small.

It is preferable that the image correction unit includes a color and/or brightness acquisition unit that acquires a color and/or brightness of the first structure from the first mode display image, and a color and/or brightness adjustment unit that performs correction for adjusting a color and/or brightness of the second mode display image such that a difference between the color and/or the brightness of the first structure in the second mode display image before the correction and the color and/or the brightness of the first structure acquired by the color and/or brightness acquisition unit becomes small.

It is preferable that the image correction unit includes a color and/or brightness storage unit that stores the color and/or the brightness of the first structure in advance, and a color and/or brightness adjustment unit that performs correction for adjusting the color and/or the brightness of the second mode display image such that a difference between the color and/or the brightness of the first structure in the second mode display image before the correction and the color and/or the brightness of the first structure stored in the color and/or brightness storage unit becomes small.

It is preferable that the observation target includes a second structure, and the image correction unit corrects the first mode display image or the second mode display image such that a difference in the color and/or the brightness of the first structure and the second structure between the first mode display image and the second mode display image before the correction becomes small. It is preferable that the image correction unit includes a structure extraction unit that extracts the second structure from the second mode display image to generate a second structure-extracted second mode display image, or extracts the first structure by removing the second structure from the second mode display image to generate a first structure-extracted second mode display image, and a color and/or brightness adjustment unit that performs correction for adjusting the color and/or the brightness of the first structure in the second mode display image by referring to the first structure-extracted second mode display image, or performs correction for adjusting the color and/or the brightness of the second structure in the second mode display image by referring to the second structure-extracted second mode display image. It is preferable that the first structure is a mucous membrane structure, and the second structure is a vascular structure.

It is preferable that the endoscope system further includes a display unit that displays the corrected image and a non-corrected image which is not corrected by the image correction unit among the first mode display image or the second mode display image, and a display control unit that controls the display unit. It is preferable that the display control unit controls the display unit such that the display unit alternately displays the non-corrected image and the corrected image. It is preferable that the display control unit has a display pattern setting unit that sets a display pattern including a display time of the non-corrected image and a display time of the corrected image on the display unit. It is preferable that the endoscope system further comprises a composite image generation unit that combines the non-corrected image and the corrected image to generate a composite image, and the display unit displays the non-corrected image, the composite image, and the corrected image.

It is preferable that the first mode illumination light is light in which a single color or a plurality of colors of light is emitted under a first mode light emission condition, and the second mode illumination light is light in which a single color or a plurality of colors of light is emitted under a second mode light emission condition that is different from the first mode light emission condition.

It is preferable that in a case where the first mode illumination light is light in which the plurality of colors of light is simultaneously emitted, the first mode display image is an image obtained by imaging the observation target that is simultaneously illuminated with the plurality of colors of light, and in a case where the first mode illumination light is light in which the plurality of colors of light is sequentially emitted, the first mode display image is an image obtained by sequentially imaging the observation target that is sequentially illuminated with the respective colors of light.

It is preferable that in a case where the second mode illumination light is light in which the plurality of colors of light is simultaneously emitted, the second mode display image is an image obtained by imaging the observation target that is simultaneously illuminated with the plurality of colors of light, and in a case where the second mode illumination light is light in which the plurality of colors of light is sequentially emitted, the second mode display image is an image obtained by sequentially imaging the observation target that is sequentially illuminated with the respective colors of light.

An endoscope system according to the invention comprises: a light source that emits first mode illumination light and second mode illumination light; an image acquisition unit that acquires a first mode display image and a second mode display image, the first mode display image being obtained by imaging an observation target including a first structure in a case where the observation target is illuminated with the first mode illumination light, and the second mode display image being obtained by imaging the observation target in a case where the observation target is illuminated with the second mode illumination light; an image correction unit that obtains a corrected image by correcting the first mode display image or the second mode display image, and performs correction such that a difference in a color and/or brightness of the first structure between the first mode display image and the second mode display image before the correction becomes small; and a display control unit that controls a display unit. The image correction unit includes a color and/or brightness acquisition unit that acquires a color and/or brightness of the first structure from the first mode display image, and a color and/or brightness adjustment unit that performs correction for adjusting a color and/or brightness of the second mode display image such that a difference between the color and/or the brightness of the first structure in the second mode display image before the correction and the color and/or the brightness of the first structure acquired by the color and/or brightness acquisition unit becomes small. The display control unit controls the display unit such that the display unit alternately displays the first mode display image, and the second mode display image of which the color and/or brightness is adjusted by the color and/or brightness adjustment unit.

A method of operating an endoscope system according to the invention comprises: a step of, by a light source, emitting first mode illumination light and second mode illumination light; a step of, by an image acquisition unit, acquiring a first mode display image and a second mode display image, the first mode display image being obtained by imaging an observation target including a first structure in a case where the first mode illumination light is emitted, and the second mode display image being obtained by imaging the observation target in a case where the second mode illumination light is emitted; and a step of, by an image correction unit, obtaining a corrected image by correcting the first mode display image or the second mode display image, and performing correction such that a difference in a color and/or brightness of the first structure between the first mode display image and the second mode display image before the correction becomes small.

According to the invention, even in a case where the normal image and the special image are alternately displayed, it is possible to reduce visual discomfort such as eye flickering.

### BRIEF DESCRIPTION OF THE DRAWINGS

Fig. 1 is an external view of an endoscope system.
Fig. 2 is a block diagram illustrating functions of an endoscope system of a first embodiment.
Fig. 3 is a graph illustrating a spectroscopic spectrum of violet light V, blue light B, blue light Bx, green light G, and red light R.
Fig. 4 is a table illustrating a pattern of illumination light emitted in a normal mode, a special mode, and a mixed mode of the first embodiment.
Fig. 5 is a block diagram illustrating functions of an image correction unit and a display control unit.
Fig. 6 is an explanatory diagram illustrating a method of generating a blood vessel-extracted special image and a mucous membrane-extracted special image.
Fig. 7 is an image diagram of a monitor alternately displaying a normal image and a corrected special image.
Fig. 8 is an explanatory diagram illustrating a flow of display of images on a monitor in a case where a display time Tc of the normal image is longer than a display time Ts of the corrected special image.
Fig. 9 is an explanatory diagram illustrating a flow of display of images on a monitor in a case where the display time Ts of the corrected special image is longer than the display time Tc of the normal image.
Fig. 10 is a flowchart illustrating a flow in a mixed mode.
Fig. 11 is a block diagram illustrating a function of an image correction unit comprising a color and/or brightness storage unit and a function of a display control unit.
Fig. 12 is a block diagram illustrating a function of an image correction unit comprising a composite image generation unit and a function of a display control unit.
Fig. 13 is an explanatory diagram illustrating a flow of display of images on a monitor in a case where a normal image, a composite image, and a corrected special image are displayed.
Fig. 14 is a table illustrating a pattern of illumination light that is emitted in a normal mode, a special mode, and a mixed mode according to a second embodiment.
Fig. 15 is a block diagram illustrating a function of an endoscope system of a third embodiment.
Fig. 16 is a graph illustrating a spectroscopic spectrum of normal mode white light.
Fig. 17 is a graph illustrating a spectroscopic spectrum of special mode white light.
Fig. 18 is a block diagram illustrating a function of an endoscope system of a fourth embodiment.
Fig. 19 is a plan view of a rotation filter.
Fig. 20 is a table illustrating a pattern of illumination light that is emitted in a normal mode, a special mode, and a mixed mode according to the fourth embodiment.
Fig. 21 is a schematic view of a capsule endoscope of a fifth embodiment.

### DESCRIPTION OF THE PREFERRED EMBODIMENTS

### [First Embodiment]

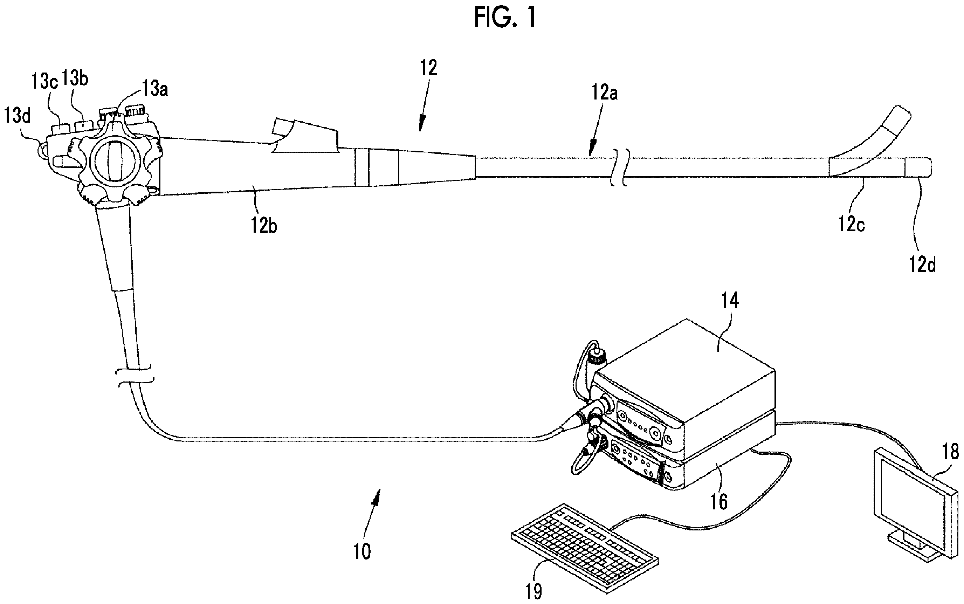

As illustrated in Fig. 1, an endoscope system 10 has an endoscope 12, a light source device 14, a processor device 16, a monitor 18 (display unit), and a console 19. The endoscope 12 is optically connected to the light source device 14, and is electrically connected to the processor device 16. The endoscope 12 has an insertion part 12a to be inserted into a subject, an operating part 12b provided at a proximal end portion of the insertion part 12a, and a bending part 12c and a distal end part 12d provided on a distal end side of the insertion part 12a. By operating an angle knob 13a of the operating part 12b, the bending part 12c makes a bending motion. The distal end part 12d is directed in a desired direction by this bending motion.

Additionally, the operating part 12b is provided with a still image acquisition unit 13b used for operating the acquisition of still images, a mode switching unit 13c used for operating the switching of observation modes, and a zooming operating unit 13d used for operating the change of a zoom magnification factor, in addition to the angle knob 13a. In the still image acquisition unit 13b, a freeze operation of displaying a still image of an observation target on the monitor 18, and a release operation of saving the still image in a storage are possible.

The endoscope system 10 has a normal mode, a special mode, and a mixed mode in which both the normal mode and the special mode are performed, as observation modes. In a case where the observation mode is the normal mode, the light source device 14 emits normal mode illumination light under a normal mode light emission condition (corresponding to a "first mode light emission condition" of the invention), and a normal image is displayed on the monitor 18 based on an image signal obtained by imaging the observation target illuminated with the normal mode illumination light.

In a case where the observation mode is the special mode, the light source device 14 emits special mode illumination light under a special mode light emission condition (corresponding to a "second mode light emission condition" of the invention) different from the normal mode light emission condition, and a special image is displayed on the monitor 18 based on an image signal obtained by imaging the observation target illuminated with the special mode illumination light. In a case where the observation mode is the mixed mode, the light source device 14 alternately emits the normal mode illumination light and the special mode illumination light, and the normal image and the special image are alternately displayed on the monitor 18.

The processor device 16 is electrically connected to the monitor 18 and the console 19. The monitor 18 outputs and displays an image of the observation target, information accompanying the image, and the like. The console 19 functions as a user interface that receives input operations, such as designation or the like of a region of interest (ROI) and function setting. The hardware structure of each unit in the processor device is various processors as described below. The various processors include a central processing unit (CPU) that is a general-purpose processor that executes software (programs) to function as various processing units, a programmable logic device (PLD) that is a processor capable of changing a circuit configuration after manufacture, such as a field programmable gate array (FPGA), and an exclusive electric circuit that is a processor having a circuit configuration exclusively designed to execute a specific process, such as an application specific integrated circuit (ASIC). In addition, the same applies the respective units inside the endoscope 12 and the light source device 14.

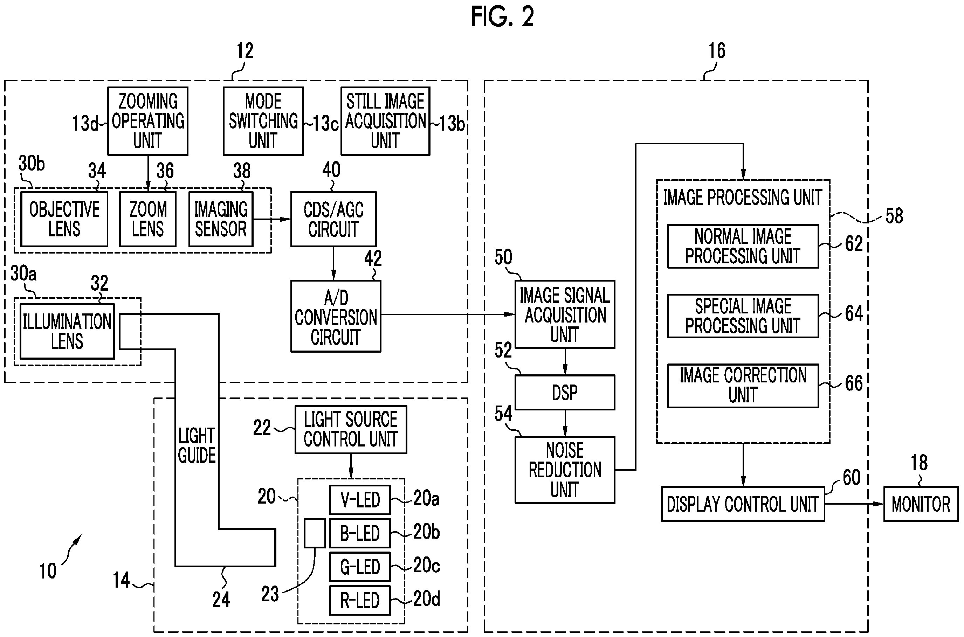

As illustrated in Fig. 2, the light source device 14 comprises a light source 20 that emits illumination light to be used for illumination of the observation target, and a light source control unit 22 that controls the light source 20. The light source 20 is a semiconductor light source, such as a plurality of colors of light emitting diodes (LEDs). The light source control unit 22 controls a quantity of light emission of the illumination light by ON/OFF of the LEDs and the adjustment of the driving currents or driving voltages of the LEDs. Additionally, the light source control unit 22 controls a wavelength range of the illumination light, for example, by changing optical filters.

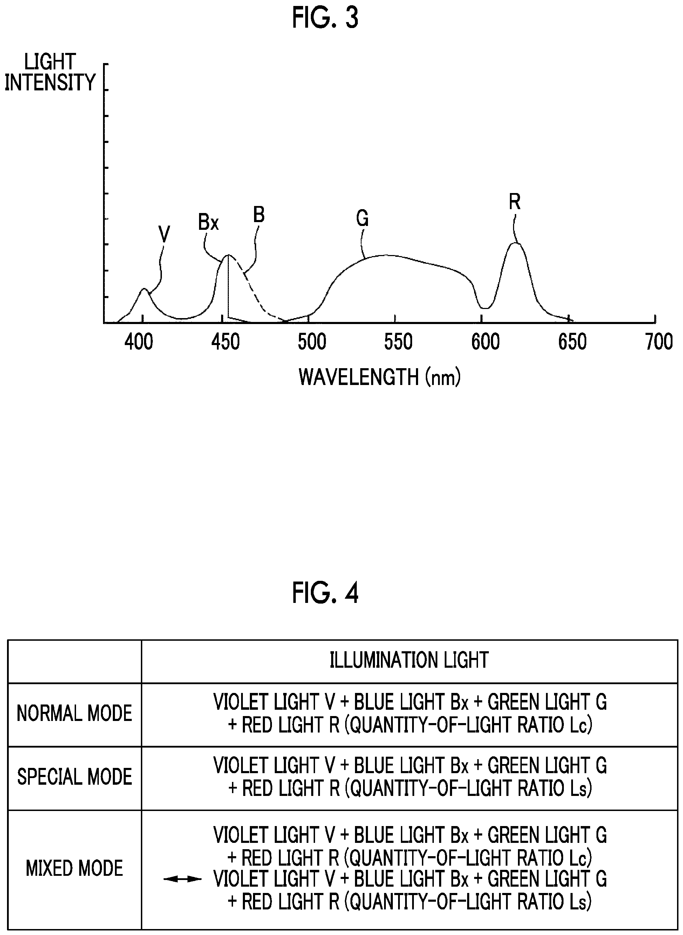

In the first embodiment, the light source 20 has four-color LEDs of a violet light emitting diode (V-LED) 20a, a blue light emitting diode (B-LED) 20b, a green light emitting diode (G-LED) 20c, and a red light emitting diode (R-LED) 20d, and a wavelength cutoff filter 23. As illustrated in Fig. 3, the V-LED 20a emits violet light V having a wavelength range of 380 nm to 420 nm.

The B-LED 20b emits blue light B having a wavelength range of 420 nm to 500 nm. Among the blue light B emitted from the B-LED 20b, at least the blue light on a longer wavelength side than a peak wavelength of 450 nm is cut by the wavelength cutoff filter 23. Accordingly, blue light Bx after being transmitted through the wavelength cutoff filter 23 has a wavelength range of 420 to 460 nm. The reason why light in a wavelength range on the longer wavelength side than 460 nm is cut is that the light in the wavelength range on the longer wavelength side than 460 nm is a factor of reducing a blood vessel contrast of a blood vessel that is the observation target. In addition, the wavelength cutoff filter 23 may reduce the light in the wavelength range on the longer wavelength side than 460 nm, instead of cutting the light in the wavelength range on the longer wavelength side than 460 nm.

The G-LED 20c emits green light G having a wavelength range of 480 nm to 600 nm. The R-LED 20d emits red light R having a wavelength range of 600 nm to 650 nm. In addition, central wavelengths and peak wavelengths of the respective colors of light emitted from the LEDs 20a to 20d may be the same as each other or may be different from each other.

The light source control unit 22 independently controls ON/OFF of the respective LEDs 20a to 20d, the quantity of light emission at the time of ON, and the like, thereby adjusting a light emission timing, a light emission period, the quantity of light, and a spectroscopic spectrum of the illumination light. The control of ON and OFF in the light source control unit 22 varies in the respective observation modes.

As illustrated in Fig. 4, in the case of the normal mode, the light source control unit 22 turns on all of the V-LED 20a, the B-LED 20b, the G-LED 20c, and the R-LED 20d. In that case, a quantity-of-light ratio Lc between the violet light V, the blue light B, the green light G, and the red light R is set such that the quantity of light emission of the blue light Bx becomes larger than the quantity of light emission of any of the violet light V, the green light G, and the red light R. Thus, in the normal mode, the normal mode illumination light including the violet light V, the blue light Bx, the green light G, and the red light R is emitted from the light source device 14. Since the normal mode illumination light has an intensity equal to or more than a given level from a blue range to a red range, the normal mode illumination light is substantially white. The normal mode light emission condition corresponds to "simultaneously emitting the violet light V, the blue light Bx, the green light G, and the red light R at the quantity-of-light ratio Lc".

Even in the case of the special mode, the light source control unit 22 turns on all of the V-LED 20a, the B-LED 20b, the G-LED 20c, and the R-LED 20d. In that case, a quantity-of-light ratio Ls between the violet light V, the blue light B, the green light G, and the red light R is set such that the quantity of light emission of the violet light V becomes larger than the quantity of light emission of any of the blue light Bx, the green light G, and the red light R, and the quantity of light emission of the green light G and the red light R becomes smaller than the quantity of light emission of the violet light V and the blue light Bx. Thus, in the special mode, the special mode illumination light including the violet light V, the blue light Bx, the green light G, and the red light R is emitted from the light source device 14. The special mode light emission condition corresponds to "simultaneously emitting the violet light V, the blue light Bx, the green light G, and the red light R at the quantity-of-light ratio Ls".

In the case of the mixed mode, the light source control unit 22 turns on all of the V-LED 20a, the B-LED 20b, the G-LED 20c, and the R-LED 20d, and switches the quantity-of-light ratio from "Lc" to "Ls", from "Ls" to "Lc" in frame units. That is, as the light emitted from the light source device 14, the normal mode illumination light and the special mode illumination light are alternately switched.

As illustrated in Fig. 2, the illumination light emitted from the light source 20 enters a light guide 24 inserted into the insertion part 12a, via a light path coupling part (not illustrated) formed by a mirror, a lens, or the like. The light guide 24 is built in the endoscope 12 and a universal cord, and propagates the illumination light up to the distal end part 12d of the endoscope 12. The universal cord is a cord that connects the endoscope 12, and the light source device 14 and the processor device 16 together. In addition, multimode fiber can be used as the light guide 24. As an example, a fine-diameter fiber cable of which the core diameter is 105 µm, the clad diameter is 125 µm, and a diameter including a protective layer used as an outer cover is φ0.3 mm to φ0.5 mm can be used for the light guide 24.

The distal end part 12d of the endoscope 12 is provided with an illumination optical system 30a and an imaging optical system 30b. The illumination optical system 30a has an illumination lens 32. The observation target is illuminated with the illumination light propagated through the light guide 24 via the illumination lens 32. The imaging optical system 30b includes an objective lens 34, a zoom lens 36, and an imaging sensor 38. Various kinds of light such as reflected light, scattered light and fluorescence from the observation target enter the imaging sensor 38 via the objective lens 34 and the zoom lens 36. Accordingly, the image of the observation target is formed on the imaging sensor 38. The zoom lens 36 freely moves between a telephoto end and a wide end by the zooming operating unit 13d being operated, and thus the image of the observation target formed on the imaging sensor 38 is magnified or reduced.

The imaging sensor 38 is a color imaging sensor that images the observation target irradiated with the illumination light. Each pixel of the imaging sensor 38 is provided with any one of a red (R) color filter, a green (G) color filter, and a blue (B) color filter. The imaging sensor 38 receives violet light to blue light with a B pixel provided with the B color filter, receives green light with a G pixel provided with the G color filter, and receives red light with an R pixel provided with the R color filter. Image signals of respective RGB colors are output from the respective color pixels. The imaging sensor 38 transmits the output image signals to a CDS/AGC circuit 40.

In the normal mode, the imaging sensor 38 images the observation target illuminated with the normal mode illumination light, thereby outputting a Bc image signal from the B pixel, outputting a Gc image signal from the G pixel, and outputting an Rc image signal from the R pixel. Additionally, in the special mode, the imaging sensor 38 images the observation target illuminated with the special mode illumination light, thereby outputting a Bs image signal from the B pixel, outputting a Gs image signal from the G pixel, and outputting an Rs image signal from the R pixel.

During the illumination with the normal mode illumination light in the mixed mode, the imaging sensor 38 images the observation target illuminated with the normal mode illumination light, thereby outputting the Bc image signal, the Gc image signal, and the Rc image signal. During the illumination with the special mode illumination light in the mixed mode, the imaging sensor 38 images the observation target illuminated with the special mode illumination light, thereby outputting the Bs image signal, the Gs image signal, and the Rs image signal.

As the imaging sensor 38, a charge coupled device (CCD) imaging sensor, a complementary metal-oxide semiconductor (CMOS) imaging sensor, or the like is available. Additionally, instead of the imaging sensor 38 provided with the color filters in the primary colors of RGB, a complementary color imaging sensor comprising complementary color filters in C (cyan), M (magenta), Y (yellow), and G (green) may be used. In a case where the complementary color imaging sensor is used, image signals of four colors of CMYG are output. For this reason, the same respective RGB image signals as those in the imaging sensor 38 can be obtained by converting the image signals of four colors of CMYG into image signals of three colors of RGB through color conversion between complementary colors and the primary colors. Additionally, instead of the imaging sensor 38, a monochrome sensor that is not provided with the color filters may be used.

The CDS/AGC circuit 40 performs correlated double sampling (CDS) and automatic gain control (AGC) on analog image signals received from the imaging sensor 38. An analog-to-digital (A/D) conversion circuit 42 converts the analog image signals, which have passed through the CDS/AGC circuit 40, into digital image signals. The A/D conversion circuit 42 inputs the digital image signals after the A/D conversion to the processor device 16.

The processor device 16 includes an image signal acquisition unit 50, a digital signal processor (DSP) 52, a noise reduction unit 54, an image processing unit 58, and a display control unit 60.

The image signal acquisition unit 50 (corresponding to the "image acquisition unit" of the invention) acquires digital image signals corresponding to the observation mode from the endoscope 12. In the normal mode, the Bc image signal, the Gc image signal, and the Rc image signal are acquired as a normal image (corresponding to the "first mode display image" of the invention). In the special mode, the Bs image signal, the Gs image signal, and the Rs image signal are acquired as a special image (corresponding to the "second mode display image" of the invention). In the mixed mode, the Bc image signal, the Gc image signal, and the Rc image signal are acquired as the normal image, and the Bs image signal, the Gs image signal, and the Rs image signal are acquired as the special image.

The DSP 52 performs various kinds of signal processing such as defect correction processing, offset processing, gain correction processing, linear matrix processing, gamma conversion processing, and demosaicing processing, on the image signals acquired by the image signal acquisition unit 50. In the defect correction processing, a signal of a defective pixel of the imaging sensor 38 is corrected. In the offset processing, a dark current component is removed from the image signals subjected to the defect correction processing, and an accurate zero level is set. In the gain correction processing, a signal level is adjusted by multiplying the image signals subjected to the offset processing by a specific gain.

The linear matrix processing enhances color reproducibility on the image signals subjected to the gain correction processing. In the gamma conversion processing, brightness and saturation of image signals subjected to the linear matrix processing are adjusted. By performing the demosaicing processing (also referred to as equalization processing or synchronization processing) on the image signals subjected to the gamma conversion processing, a signal of a color that runs short in each pixel is generated by interpolation. By means of the demosaicing processing, all pixels have signals of respective RGB colors. The noise reduction unit 54 performs noise reducing processing using, for example, a moving average method or a median filter method on the image signals subjected to the demosaicing processing or the like in the DSP 52, and reduces noise.

The image processing unit 58 includes a normal image processing unit 62, a special image processing unit 64, and an image correction unit 66. The normal image processing unit 62 operates in a case where the normal mode or the mixed mode is set, and performs color conversion processing, color enhancement processing, and structure enhancement processing on the received normal image. In the color conversion processing, color conversion processing is performed on the RGB image signals by 3x3 matrix processing, gradation transformation processing, three-dimensional look-up table (LUT) processing, and the like.

The color enhancement processing is performed on the image signals subjected to the color conversion processing. The structure enhancement processing is processing of enhancing the structure of the observation target, and is performed on the image signals after the color enhancement processing. As described above, since the normal image subjected to the various kinds of image processing up to the structure enhancement processing is an image obtained on the basis of the normal mode illumination light in which the violet light V, the blue light Bx, the green light G, and the red light R are emitted in a well-balanced manner, the normal image is a natural-tone image. The normal image is input to the display control unit 60 as it is in a case where the normal mode is set, and is input to the display control unit 60 and the image correction unit 66 in a case where the mixed mode is set.

The special image processing unit 64 operates in a case where the special mode or the mixed mode is set. In the special image processing unit 64, the color conversion processing, the color enhancement processing, and the structure enhancement processing are performed on the received special image. The processing contents of the color conversion processing, the color enhancement processing, and the structure enhancement processing are the same as those of the normal image processing unit 62. Since the special image is an image obtained on the basis of the special mode illumination light in which the violet light V with a high absorption coefficient of hemoglobin of blood vessels has a larger quantity of light emission than the blue light Bx, the green light G, and the red light R in the other colors, the resolution of a vascular structure is higher than that of the other structures. The special image is input to the display control unit 60 as it is in a case where the special mode is set, and is input to the image correction unit 66 in a case where the mixed mode is set.

The image correction unit 66 operates in a case where the mixed mode is set, and performs correction on the special image. The image correction unit 66 corrects the special image such that the difference between the color and/or the brightness of the vascular structure or the mucous membrane structure in the special image before correction and the color and/or the brightness of the mucous membrane structure in the normal image becomes small. The details of the image correction unit 66 will be described later. The corrected special image obtained by correcting the special image by the image correction unit 66 is transmitted to the display control unit 60. Note that the image correction unit 66 may perform correction such that both the difference in color and the difference in brightness become small, or may perform correction such that either the difference in color or the difference in brightness becomes small.

The display control unit 60 performs a display control for displaying an image from the image processing unit 58 on the monitor 18. In a case where the normal mode is set, the display control unit 60 performs the control of displaying the normal image on the monitor 18. In a case where the special mode is set, the display control unit 60 performs the control of displaying the special image on the monitor 18. In a case where the mixed mode is set, the normal image and the corrected special image are displayed according to the set display pattern. The details of the display control in the mixed mode will be described later.

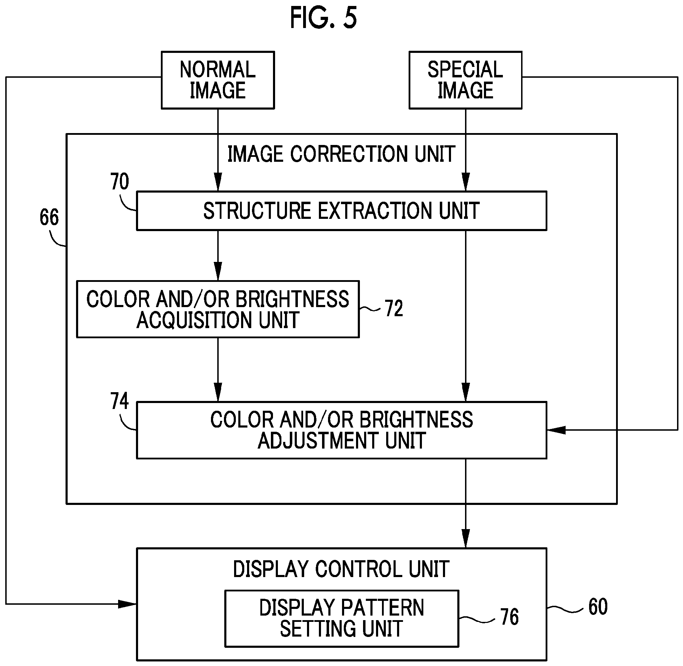

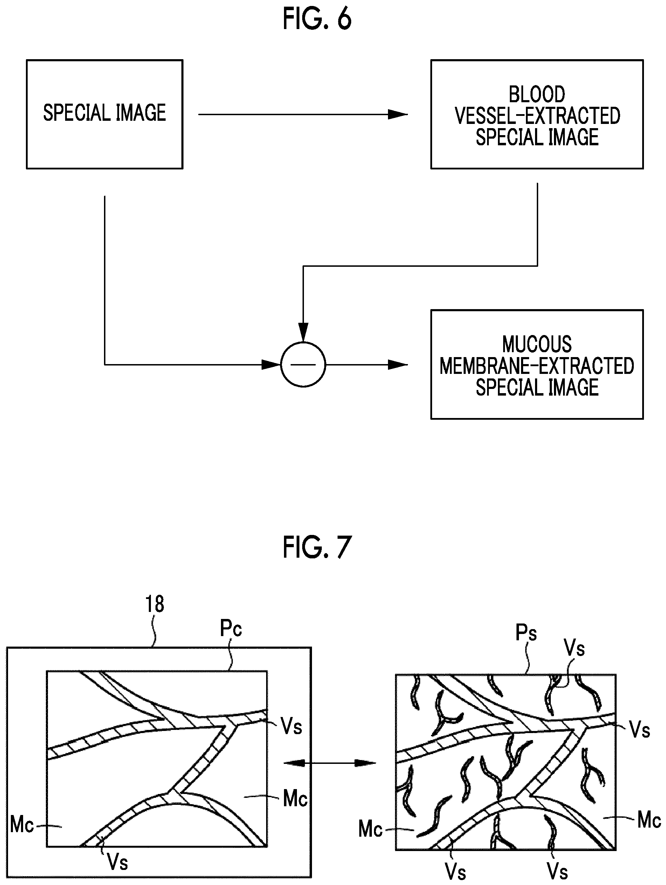

As illustrated in Fig. 5, the image correction unit 66 includes a structure extraction unit 70, a color and/or brightness acquisition unit 72, and a color and/or brightness adjustment unit 74. As illustrated in Fig. 6, the structure extraction unit 70 extracts a vascular structure from the special image to generate a blood vessel-extracted special image. As a method of extracting the vascular structure, for example, in addition to a method of binarizing a blood vessel and other parts using a predetermined threshold, a method of extracting blood vessels by obtaining a ratio of or a difference between a Bs image signal and a Gs image signal of the special image is considered. Similarly, the structure extraction unit 70 extracts the vascular structure from the normal image to generate a blood vessel-extracted normal image.

In a case where it is difficult to extract the vascular structure from the normal image, a structure on the normal image at the same position as the vascular structure extracted from the special image may be extracted as the vascular structure. However, in a case where all the structures at the same position as the vascular structure of the special image are regarded as the vascular structure, there is a possibility that a structure which is not visible in the normal image may be extracted as a vascular structure. Therefore, it is preferable to extract a structure having a low resolution that can be visually recognized in the normal image, and at the same position on the normal image, among the vascular structure of the special image, as the vascular structure.

The structure extraction unit 70 performs difference processing between the special image and the blood vessel-extracted special image, to generate a blood vessel-removed special image which is obtained by removing the vascular structure from the special image. The blood vessel-removed special image is a mucous membrane-extracted special image in which the vascular structure is removed and almost only the mucous membrane structure is extracted. As described above, the reason why the mucous membrane-extracted special image is generated by the difference processing between the special image and the blood vessel-extracted special image is that it is difficult to extract only the mucous membrane structure because the mucous membrane structure does not have much difference in pixel value from other parts. Similarly, the structure extraction unit 70 performs the difference processing between the normal image and the blood vessel-extracted normal image, to generate a blood vessel-removed normal image which is obtained by removing the vascular structure from the normal image (mucous membrane-extracted normal image).

The color and/or brightness acquisition unit 72 acquires the color and/or the brightness of the vascular structure from the blood vessel-extracted normal image. In addition, the color and/or brightness acquisition unit 72 acquires the color and/or the brightness of the mucous membrane structure from the mucous membrane-extracted normal image. It is preferable to average the acquired color of the vascular structure or the mucous membrane structure, and it is also preferable to average the acquired brightness of the vascular structure or the mucous membrane structure. Here, as a method of acquiring the color or brightness, for example, a method of performing processing of separating the color and the brightness on the blood vessel-extracted normal image is considered. In a case where the blood vessel-extracted normal image is converted into a luminance signal Y and color-difference signals Cr and Cb, the luminance signal Y corresponds to the brightness of the vascular structure, and the color-difference signals Cr and Cb correspond to the color of the vascular structure. It is preferable that the color and/or brightness acquisition unit 72 acquires at least one of the color or brightness of the vascular structure.

The color and/or brightness adjustment unit 74 performs correction for adjusting the color and/or the brightness of the special image such that the difference between the color and/or the brightness of the vascular structure in the special image before correction and the average value of the color and/or the brightness of the vascular structure acquired by the color and/or brightness acquisition unit 72 becomes small by referring to the position of the vascular structure in the blood vessel-extracted special image. In addition, the color and/or brightness adjustment unit 74 performs correction for adjusting the color and/or the brightness of the special image such that the difference between the color and/or the brightness of the mucous membrane structure in the special image before correction and the average value of the color and/or the brightness of the mucous membrane structure acquired by the color and/or brightness acquisition unit 72 becomes small by referring to the position of the mucous membrane structure in the mucous membrane-extracted special image. Thereby, a corrected special image in which the color and/or the brightness of the vascular structure or the mucous membrane structure is similar to the color and/or the brightness of the normal image is obtained.

Note that "the difference in color and/or brightness becomes small" means that the color and/or the brightness of the vascular structure or the mucous membrane structure in the special image matches the average value of the color and/or the brightness of the vascular structure or the mucous membrane structure acquired by the color and/or brightness acquisition unit 72, and also that the color and/or the brightness of the vascular structure or the mucous membrane structure in the special image is within a certain range including the average value of the color and/or the brightness of the vascular structure or the mucous membrane structure acquired by the color and/or brightness acquisition unit 72.

As illustrated in Fig. 7, the display control unit 60 performs a control of outputting a display instruction to the monitor 18 such that the monitor 18 alternately displays a normal image Pc (corresponding to the "non-corrected image" of the invention) and a corrected special image Ps (corresponding to the "corrected image" of the invention). Since the normal image Pc and the corrected special image Ps are alternately displayed on the monitor 18, it is possible to observe both the normal image Pc and the corrected special image Ps without operating the mode switching unit 13c. A vascular structure Vs that cannot be observed in the normal image Pc can also be observed in the corrected special image Ps, and such a vascular structure Vs contributes to effective diagnosis. Even in a case where the normal image Pc and the corrected special image Ps are alternately displayed, since the difference between the color and/or the brightness of a mucous membrane structure Mc or the vascular structure Vs, which occupies most of the corrected special image Ps, and the color and/or the brightness of the normal image Pc is small, there is no fear that visual discomfort such as eye flickering will occur.

Further, the display control unit 60 has a display pattern setting unit 76 for setting display patterns of the normal image and the corrected special image to be displayed on the monitor 18. The setting of the display pattern is performed by operating the display pattern setting unit 76 by using an input device such as the console 19. The display pattern setting unit 76 sets a display time Tc of the normal image and a display time Ts of the corrected special image as the display pattern. The display control unit 60 outputs a display instruction to the monitor 18 such that the monitor 18 displays the normal image and the corrected special image according to the display time set by the display pattern setting unit 76.

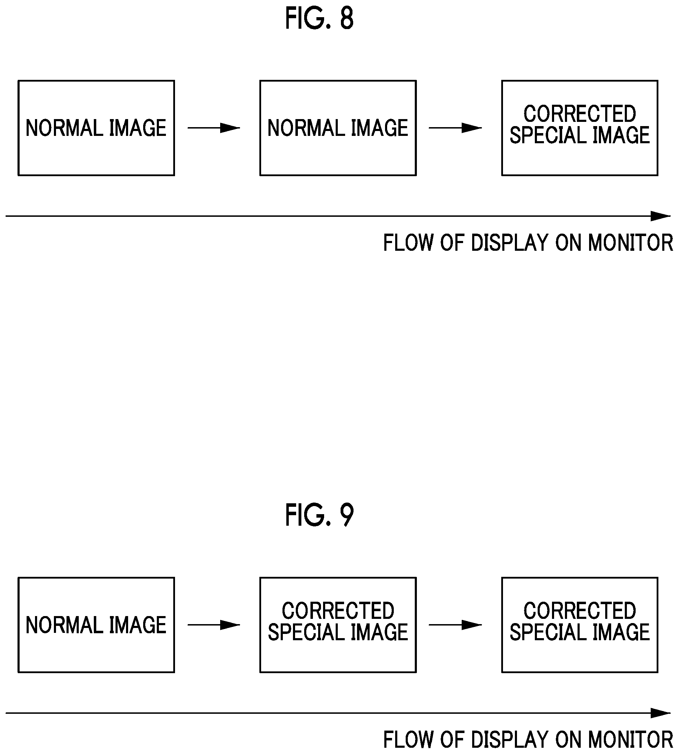

For example, in a case where the display time Tc of the normal image is set longer than the display time Ts of the corrected special image, as illustrated in Fig. 8, a display pattern in which the corrected special image for one frame is displayed after the normal image for several frames is displayed is set. This display pattern is suitable for a case where the lesion part is not overlooked while the entire observation target is observed, as in screening. On the other hand, in a case where the display time Ts of the corrected special image is set longer than the display time Tc of the normal image, as illustrated in Fig. 9, a display pattern in which the corrected special image for several frames is displayed after the normal image for one frame is displayed is set. This display pattern is suitable for a case where the properties of the lesion part are observed in detail, as in the detailed observation.

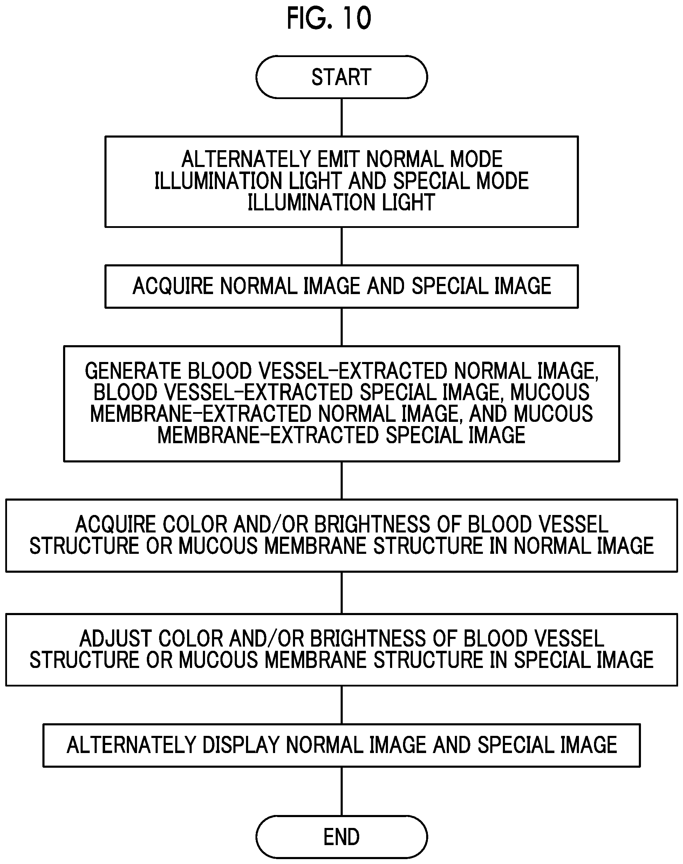

Next, a series of flows in the mixed mode will be described with reference to the flowchart of Fig. 10. The mode is switched to the mixed mode by operating the mode switching unit 13c. As a result, the normal mode illumination light and the special mode illumination light are alternately emitted. That is, as the normal mode illumination light, the violet light V, the blue light Bx, the green light G, and the red light R are simultaneously emitted at the quantity-of-light ratio Lc, and thereafter, as the special mode illumination light, the violet light V, the blue light Bx, the green light G, and the red light R are simultaneously emitted at the quantity-of-light ratio Ls.

Then, the observation target illuminated with the violet light V, the blue light Bx, the green light G, and the red light R at the quantity-of-light ratio Lc is imaged by the imaging sensor 38, and the normal image constituted of the Bc image signal, the Gc image signal, and the Rc image signal is obtained. In addition, the observation target illuminated with the violet light V, the blue light Bx, the green light G, and the red light R at the quantity-of-light ratio Ls is imaged by the imaging sensor 38, and the special image constituted of the Bs image signal, the Gs image signal, and the Rs image signal is obtained.

In a case where the normal image and the special image are generated, the normal image and the special image are input to the image correction unit 66. The structure extraction unit 70 extracts the vascular structure from the special image to generate the blood vessel-extracted special image, and extracts the mucous membrane structure by removing the vascular structure from the special image to generate the mucous membrane-extracted special image. Similarly, the structure extraction unit 70 extracts the vascular structure from the normal image to generate the blood vessel-extracted normal image, and extracts the mucous membrane structure by removing the vascular structure from the normal image to generate the mucous membrane-extracted normal image.

Next, the color and/or brightness acquisition unit 72 acquires the average value of the color and/or the brightness of the vascular structure from the blood vessel-extracted normal image, and acquires the average value of the color and/or the brightness of the mucous membrane structure from the mucous membrane-extracted normal image. Then, the color and/or brightness adjustment unit 74 performs correction for adjusting the color and/or the brightness of the special image such that the difference between the color and/or the brightness of the vascular structure in the special image before correction and the color and/or the brightness of the vascular structure acquired by the color and/or brightness acquisition unit 72 becomes small by referring to the position of the vascular structure in the blood vessel-extracted special image. In addition, the color and/or brightness adjustment unit 74 performs correction for adjusting the color and/or the brightness of the special image is performed such that the difference between the color and/or the brightness of the mucous membrane structure in the special image before correction and the color and/or the brightness of the mucous membrane structure acquired by the color and/or brightness acquisition unit 72 becomes small by referring to the position of the mucous membrane structure in the mucous membrane-extracted special image. As described above, correction for adjusting the color and/or the brightness of the vascular structure or the mucous membrane structure is performed, and thereby a corrected special image is obtained.

Next, the display control unit 60 performs control such that the monitor 18 alternately displays the normal image and the corrected special image according to the specific display pattern. In the display control by the display control unit 60, the display pattern setting unit 76 adjusts the display time Tc of the normal image and the display time Ts of the corrected special image as the specific display pattern.

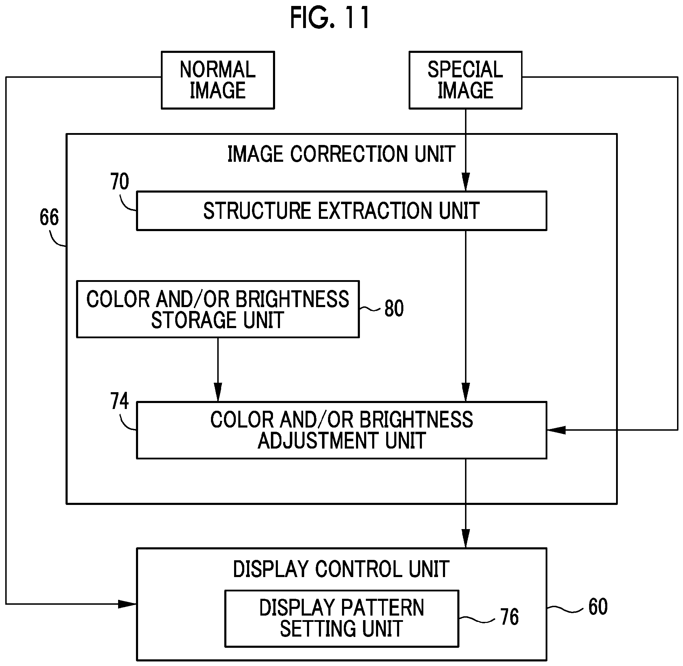

In the above embodiment, the color and/or the brightness of the vascular structure or the mucous membrane structure is acquired from the normal image, and the special image is corrected such that the color and/or the brightness of the vascular structure or the mucous membrane structure in the special image becomes the acquired color and/or brightness of the vascular structure or the mucous membrane structure; however, instead of this, the average color and/or brightness of the vascular structure or the mucous membrane structure is stored in advance, and the special image may be corrected such that the color and/or the brightness of the vascular structure or the mucous membrane structure in the special image becomes the stored color and/or brightness of the vascular structure or the mucous membrane structure. In this case, as illustrated in Fig. 11, the image correction unit 66 is provided with a color and/or brightness storage unit 80 instead of the color and/or brightness acquisition unit 72. With the color and/or brightness storage unit 80 being provided, it is not necessary for the structure extraction unit 70 to acquire the blood vessel-extracted normal image and the mucous membrane-extracted normal image.

The color and/or brightness storage unit 80 stores the average value of the color and/or the brightness of the vascular structure or the mucous membrane structure. It is preferable that the average value of the color and/or the brightness of the vascular structure or the mucous membrane structure stored in the color and/or brightness storage unit 80 is calculated from the normal image which is obtained by imaging a predetermined part (esophagus, stomach, and large intestine) in advance in the normal mode. In addition, the average value of the color and/or the brightness of the vascular structure or the mucous membrane structure may be stored for each part such as the stomach or large intestine, and depending on the part to be observed, the average value of the color and/or the brightness of the vascular structure or the mucous membrane structure used in the color and/or brightness adjustment unit 74 may be switched.

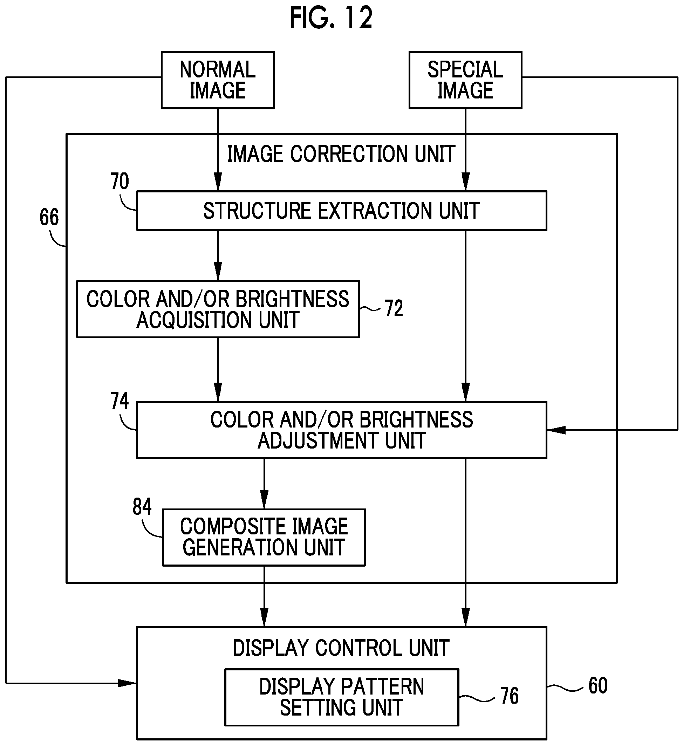

In the above embodiment, the normal image and the corrected special image are alternately displayed; however, in addition to the normal image and the corrected special image, a composite image which is obtained by combining the normal image and the corrected special image may be displayed between the normal image and the corrected special image. In this case, as illustrated in Fig. 12, the image processing unit 58 is provided with a composite image generation unit 84 for combining the normal image and the corrected special image. The composite image generation unit 84 generates a composite image by adding specific weights to the normal image and the corrected special image.

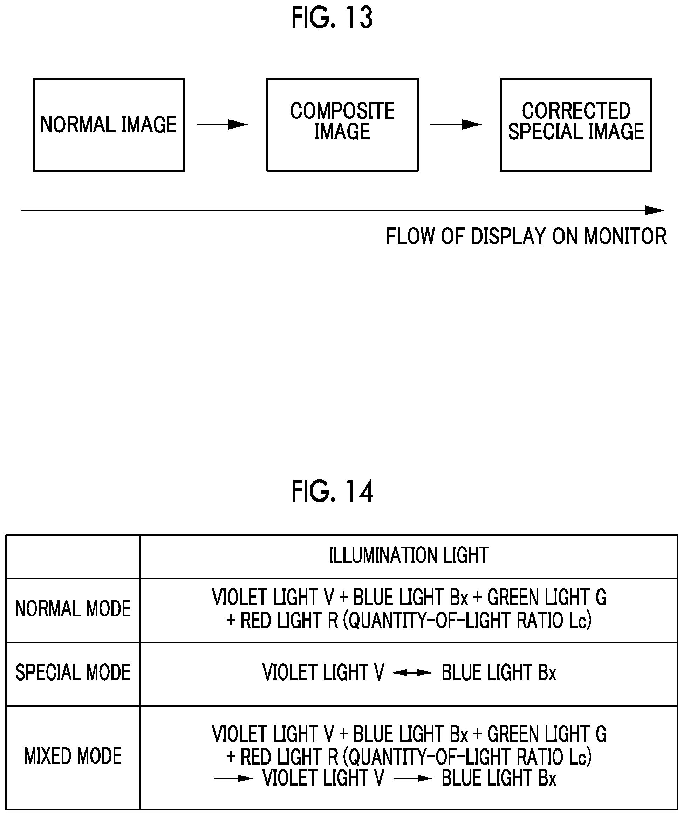

By setting the specific weighting coefficients to roughly intermediate values, the composite image is an image of which the color and/or brightness is close to the color and/or the brightness of either the normal image or the corrected special image. As illustrated in Fig. 13, the generated composite image is displayed between the normal image and the corrected special image. As described above, in a case where the composite image is displayed between the normal image and the corrected special image, a change in color and/or brightness becomes gentle as compared with a case where the normal image and the corrected special image are alternately displayed, and thus the visual discomfort such as eye flickering can be further reduced.

In the above embodiment, the image correction unit 66 corrects the special image, but on the contrary, the image correction unit 66 may correct the normal image. In this case, correction for adjusting the color and/or the brightness of the normal image may be performed such that the difference between the color and/or the brightness of the vascular structure or the mucous membrane structure in the normal image before correction and the color and/or the brightness of the vascular structure or the mucous membrane structure of the special image becomes small. In a case where the normal image is corrected, a corrected normal image (corresponding to the "corrected image" of the invention) and a special image (corresponding to the "non-corrected image" of the invention) are alternately displayed.

The color and/or brightness adjustment unit 74 performs correction for adjusting the color and/or the brightness of both the vascular structure and the mucous membrane structure, but may adjust the color and/or the brightness of at least one of the vascular structure or the mucous membrane structure. In addition, instead of adjusting both the color and the brightness, at least one of the color or the brightness may be adjusted.

### [Second Embodiment]

In the first embodiment, in the special mode, all the violet light V, the blue light Bx, the green light G, and the red light R are simultaneously emitted as the special mode illumination light, but in the second embodiment, as illustrated in Fig. 14, in the special mode, the violet light V and the blue light Bx are alternately emitted as the special mode illumination light. In the mixed mode, as the normal mode illumination light, the violet light V, the blue light Bx, the green light G, and the red light R are simultaneously emitted at the quantity-of-light ratio Lc, and thereafter, as the special mode illumination light, the violet light V and the blue light Bx are alternately emitted.

In the second embodiment, the special mode light emission condition corresponds to "alternately emitting the violet light V and the blue light Bx". Further, in the second embodiment, a plurality of colors of light is simultaneously emitted for the normal mode illumination light, and a plurality of colors of light is sequentially emitted for the special mode illumination light such that the violet light V and the blue light Bx are alternately emitted. On the contrary, a plurality of colors of light may be sequentially emitted for the normal mode illumination light, and a plurality of colors of light may be simultaneously emitted for the special mode illumination light.

In the second embodiment, in a case where the special mode illumination light is emitted in the special mode or the mixed mode, the image signal output from the imaging sensor 38 is different from that in the first embodiment. In a case where the violet light V and the blue light Bx are alternately emitted as the special mode illumination light, the observation target in a case where the violet light V is emitted is imaged by the imaging sensor 38, and the imaging sensor 38 outputs a B1 image signal from the B pixel, outputs a G1 image signal from the G pixel, and outputs an R1 image signal from the R pixel. Also, the observation target in a case where the blue light Bx is emitted is imaged by the imaging sensor 38, and the imaging sensor 38 outputs a B2 image signal from the B pixel, outputs a G2 image signal from the G pixel, and outputs an R2 image signal from the R pixel.

In the second embodiment, in a case where the special mode illumination light is emitted in the special mode or the mixed mode, the image signal acquired by the image signal acquisition unit 50 is different from that in the first embodiment, and the contents of processing in the special image processing unit 64 are different from those in the first embodiment. Among the image signals obtained by the special mode illumination light, the B1 image signal and the B2 image signal are acquired as the special image, and other image signals (the G1 image signal, the R1 image signal, the G2 image signal, and the R2 image signal) are deleted or the like.

The special image processing unit 64 generates a special image based on the B1 image signal and the B2 image signal. For example, in a case where the special image is configured of the luminance signal Y and the color-difference signals Cr and Cb, it is preferable to assign the B1 image signal or the B2 image signal to the luminance signal Y. It is preferable to assign, to the color-difference signals Cr and Cb, the resultant obtained by multiplying the calculated image of the ratio of or the difference between the B1 image signal and the B2 image signal by a predetermined coefficient. In the second embodiment, it is preferable that the special image processing unit 64 performs color conversion processing, color enhancement processing, and structure enhancement processing on the special image.

### [Third Embodiment]

In a third embodiment, the observation target is illuminated using a laser light source and a fluorescent body instead of the four-color LEDs 20a to 20d illustrated in the first embodiment. Except for that, the third embodiment is the same as the first embodiment.

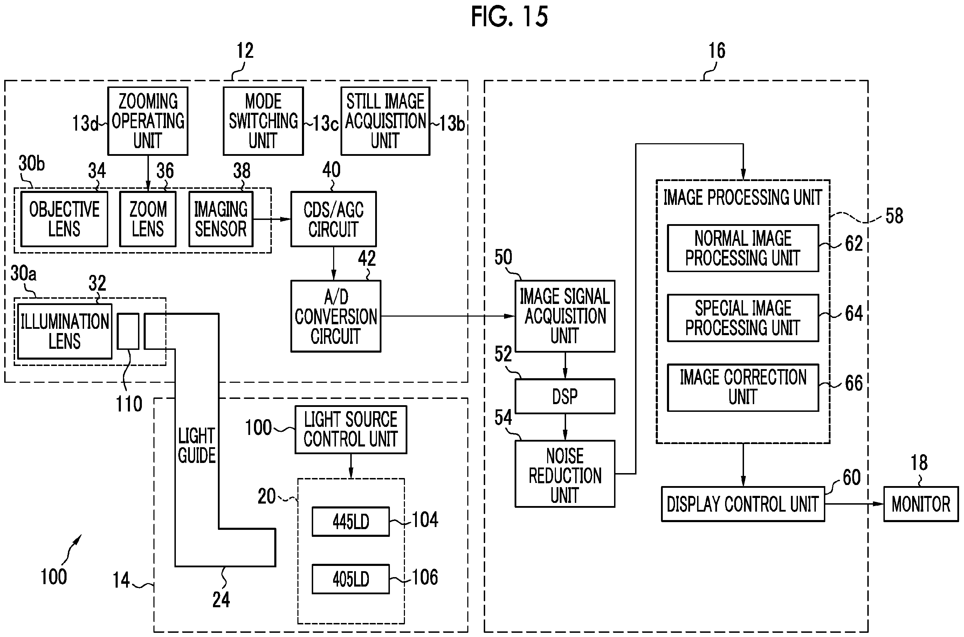

As illustrated in Fig. 15, in the endoscope system 100 of the third embodiment, the light source 20 of the light source device 14 is provided with a blue laser light source (written as "445LD"; LD represents the "laser diode") 104 that emits blue laser light having a central wavelength of 445 ± 10 nm and a blue-violet laser light source (written as "405LD") 106 that emits blue-violet laser light having a central wavelength of 405 ± 10 nm, instead of the four-color LEDs 20a to 20d. The light emission from semiconductor light-emitting elements of the respective light sources 104 and 106 are individually controlled by a light source control unit 108, and the quantity-of-light ratio of the emitted light of the blue laser light source 104 and the emitted light of the blue-violet laser light source 106 is changeable.

In the normal mode, the light source control unit 108 turns on the blue laser light source 104. In contrast, in the special mode, the light source control unit 108 turns on both the blue laser light source 104 and the blue-violet laser light source 106, and controls the emission ratio of the blue laser light to be larger than the emission ratio of the blue-violet laser light. In the mixed mode, control is performed such that the blue laser light source 104 and the blue-violet laser light source 106 are alternately turned on. The laser light emitted from the light sources 104 and 106 enters the light guide 24.

In addition, it is preferable that the half width of the blue laser light or the blue-violet laser light is about ±10 nm. Additionally, as the blue laser light source 104 and the blue-violet laser light source 106, broad area type InGaN-based laser diodes can be utilized, and InGaNAs-based laser diodes and GaNAsb-based laser diodes can also be used. Additionally a configuration using a light emitter, such as a light emitting diode, may be adopted as the light source.

In addition to the illumination lens 32, a fluorescent body 110 that blue laser light or blue-violet laser light from the light guide 24 enters is provided in the illumination optical system 30a. The fluorescent body 110 is excited by the blue laser light to emit fluorescence. In addition, a portion of the blue laser light is transmitted through the fluorescent body 110 without exciting the fluorescent body 110. The blue-violet laser light is transmitted through the fluorescent body 110 without exciting the fluorescent body 110. The inside of the body of the observation target is illuminated with the light emitted from the fluorescent body 110 via the illumination lens 32.

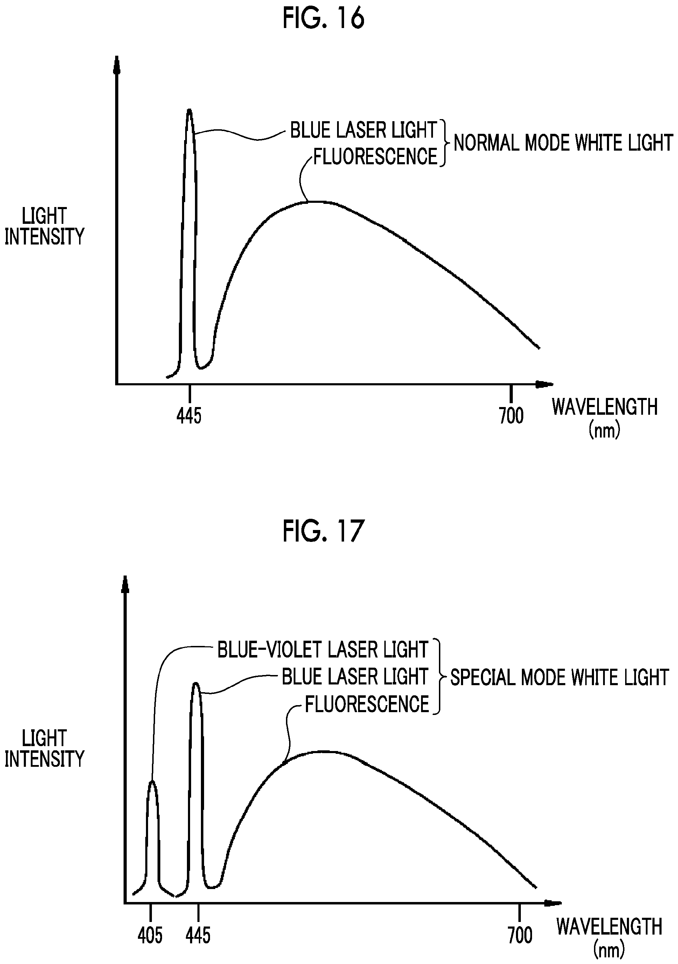

Here, in the normal mode, since the blue laser light mainly enters the fluorescent body 110, the observation target is illuminated with the normal mode white light (corresponding to the "normal mode illumination light" of the invention), which is obtained by combining the blue laser light and the fluorescence excited and emitted from the fluorescent body 110 due to the blue laser light as illustrated in Fig. 16. In the special mode, since both the blue-violet laser light and the blue laser light enter the fluorescent body 110, the observation target is illuminated with the special mode white light (corresponding to the "special mode illumination light" of the invention), which is obtained by combining the blue-violet laser light, the blue laser light, and the fluorescence excited and emitted from the fluorescent body 110 due to the blue laser light as illustrated in Fig. 17.

In the mixed mode, since the blue-violet laser light and the blue laser light alternately enter the fluorescent body 110, the observation target is alternately illuminated with the normal mode white light and the special mode white light. In the third embodiment, the normal mode light emission condition corresponds to "emitting normal mode white light", and the special mode light emission condition corresponds to "emitting special mode white light".

In addition, as the fluorescent body 110, it is preferable to use those configured to include a plurality of types of fluorescent bodies (for example, a YAG-based fluorescent body or a fluorescent body such as BAM (BaMgAl₁₀O₁₇)) that absorb a portion of the blue laser light and are excited to emit light in green to yellow. As in the configuration example, in a case where the semiconductor light-emitting elements are used as the excitation light sources of the fluorescent body 110, high-intensity white light is obtained with a high luminous efficiency, the intensity of the white light can be easily adjusted, and changes in color temperature and chromaticity of the white light can be suppressed to be small.

### [Fourth Embodiment]

In the fourth embodiment, the observation target is illuminated using a broadband light source such as a xenon lamp, and the rotation filter, instead of the four-color LEDs 20a to 20d. In addition, the observation target may be imaged by a monochrome imaging sensor instead of the color imaging sensor 38. Except for that, the fourth embodiment is the same as the first embodiment.

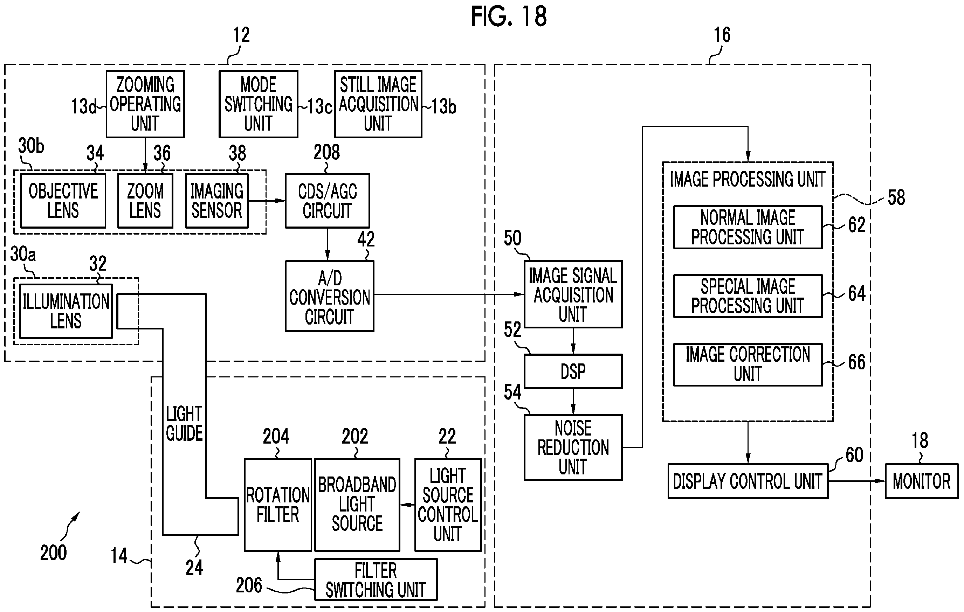

In an endoscope system 200 illustrated in Fig. 18, instead of the respective LEDs 20a to 20d of the endoscope system 10, a broadband light source 202, a rotation filter 204, and a filter switching unit 206 are provided in the light source device 14. In addition, the imaging optical system 30b is provided with a monochrome imaging sensor 208 which is not provided with a color filter, instead of the color imaging sensor 38.

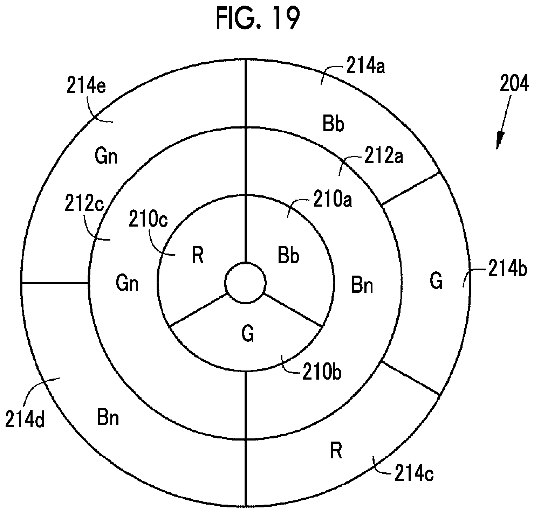

The broadband light source 202 is a xenon lamp, a white LED, or the like, and emits broadband light having a wavelength range from blue to red. The rotation filter 204 comprises a normal mode filter 210 provided on the inner side closest to the rotation axis, a special mode filter 212 provided on the outside the normal mode filter 210, and a mixed mode filter 214 provided on the outside the special mode filter 212 (refer to Fig. 19).

The filter switching unit 206 moves the rotation filter 204 in a radial direction. Specifically, the filter switching unit 206 inserts the normal mode filter 210 into a broadband light path in a case where the normal mode is set by the mode switching unit 13c. The filter switching unit 206 inserts the special mode filter 212 into the broadband light path in a case where the special mode is set. The filter switching unit 206 inserts the mixed mode filter 214 into the broadband light path in a case where the mixed mode is set.

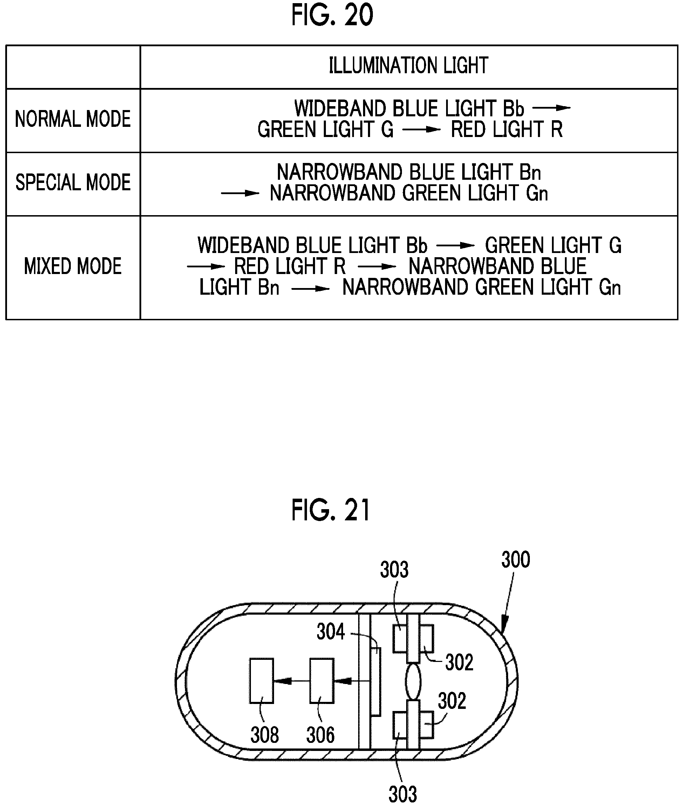

As illustrated in Fig. 19, a Bb filter 210a, a G filter 210b, and an R filter 210c are provided in the circumferential direction in the normal mode filter 210. The Bb filter 210a transmits the broadband blue light Bb having a wavelength range of 400 to 500 nm among the broadband light. The G filter 210b transmits the green light G among the broadband light. The R filter 210c transmits the red light R among the broadband light. Accordingly, in the normal mode, as the rotation filter 204 rotates, the broadband blue light Bb, the green light G, and the red light R are sequentially emitted, as the normal mode illumination light, toward the observation target, as illustrated in Fig. 20. In the fourth embodiment, the normal mode light emission condition corresponds to "sequentially emitting broadband blue light Bb, green light G, and red light R".

A Bn filter 212a and a Gn filter 212b are provided in the circumferential direction in the special mode filter 212. The Bn filter 212a transmits the narrowband blue light Bn of 400 to 450 nm among the broadband light. The Gn filter 212b transmits the narrowband green light Gn of 530 to 570 nm among the broadband light. Therefore, in the special mode, as the rotation filter 204 rotates, the narrowband blue light and the narrowband green light are sequentially emitted, as the special mode illumination light, toward the observation target. In the fourth embodiment, the special mode light emission condition corresponds to "sequentially emitting narrowband blue light and narrowband green light".

A Bb filter 214a, a G filter 214b, an R filter 214c, a Bn filter 214d, and a Gn filter 214e are provided in the circumferential direction in the mixed mode filter 214. The Bb filter 214a, the G filter 214b, the R filter 214c, the Bn filter 214d, and the Gn filter 214e have the same transmittance as the Bb filter 210a, the G filter 210b, the R filter 210c, the Bn filter 212a, and the Gn filter 212b, respectively. Therefore, in the mixed mode, as the rotation filter 204 rotates, the broadband blue light Bb, the green light G, and the red light R are sequentially emitted, as the normal mode illumination light, toward the observation target, and the narrowband blue light Bn and the narrowband green light Gn are sequentially emitted, as the special mode illumination light, toward the observation target.

In the endoscope system 200, in the normal mode, the observation target is imaged by the monochrome imaging sensor 208 whenever the observation target is illuminated with the broadband blue light Bb, the green light G, and the red light R. As a result, the Bc image signal is obtained at the time of the illumination with the broadband blue light Bb, the Gc image signal is obtained at the time of the illumination with the green light G, and the Rc image signal is obtained at the time of the illumination with the red light R. The normal image is constituted of the Bn image signal, the Gc image signal, and the Rc image signal.

In the special mode, the observation target is imaged by the monochrome imaging sensor 208 whenever the observation target is illuminated with the narrowband blue light Bn and the narrowband green light Gn. As a result, the Bn image signal is obtained at the time of the illumination with the narrowband blue light Bn, and the Gn image signal is obtained at the time of the illumination with the narrowband green light Gn. The special image is constituted of the Bn image signal and the Gn image signal.

In the mixed mode, the observation target is imaged by the monochrome imaging sensor 208 whenever the observation target is illuminated with the broadband blue light Bb, the green light G, the red light R, the narrowband blue light Bn, and the narrowband green light Gn. As a result, the Bc image signal, the Gc image signal, the Rc image signal, the Bn image signal, and the Gn image signal are obtained. An image constituted of the Bc image signal, the Gc image signal, and the Rc image signal corresponds to the normal image, and an image constituted of the Bn image signal and the Gn image signal corresponds to the special image.

### [Fifth Embodiment]

In the first to fourth embodiments, the observation is performed by inserting the endoscope 12 provided with the imaging sensor 38 into the subject, but instead of this, in the fifth embodiment, the observation for the inside of the subject is performed by a capsule endoscope which is swallowed by the patient from the mouth.

A capsule endoscope system has at least, for example, a capsule endoscope 300 illustrated in Fig. 21 and a processor device (not illustrated). The capsule endoscope 300 comprises a light source 302, a light source control unit 303, an imaging sensor 304, an image signal processing unit 306, and a transmission and reception antenna 308. The light source 302 is configured similarly to the light source 20 of the endoscope system 10. The light source control unit 303 controls driving of the light source 302. In the fifth embodiment, since the mixed mode is always set, the light source control unit 303 controls the light source 302 such that the light source 302 alternately emits the normal mode illumination light and the special mode illumination light.

The imaging sensor 304 is constituted of a color imaging sensor, similarly to the imaging sensor 38 of the endoscope system 10. The image signal processing unit 305 performs the same processing as the CDS/AGC circuit 40 and the A/D conversion circuit 42 on the image signal output from the imaging sensor 304. The image signal that has passed through the image signal processing unit 306 is wirelessly transmitted to the processor device (not illustrated) of the capsule endoscope system via the transmission and reception antenna 308. The processor device of the capsule endoscope system is configured similarly to the processor device 16 of the endoscope system 10 and also functions as an image processing unit 58. In this processor device, the same processing as in the first embodiment is performed based on the received image signal.

### Explanation of References

- 10:: endoscope system
- 12:: endoscope
- 12a:: insertion part
- 12b:: operating part
- 12c:: bending part
- 12d:: distal end part
- 13a:: angle knob
- 13b:: still image acquisition unit
- 13c:: mode switching unit
- 13d:: zooming operating unit
- 14:: light source device
- 16:: processor device
- 18:: monitor
- 19:: console
- 20:: light source
- 20a:: V-LED
- 20b:: B-LED
- 20c:: G-LED
- 20d:: R-LED
- 22:: light source control unit
- 23:: wavelength cutoff filter
- 24:: light guide
- 30a:: illumination optical system
- 30b:: imaging optical system
- 32:: illumination lens
- 34:: objective lens
- 36:: zoom lens
- 38:: imaging sensor
- 40:: CDS/AGC circuit
- 42:: A/D conversion circuit
- 50:: image signal acquisition unit
- 52:: DSP
- 54:: noise reduction unit
- 58:: image processing unit
- 60:: display control unit
- 62:: normal image processing unit
- 64:: special image processing unit
- 66:: image correction unit
- 70:: structure extraction unit
- 72:: color and/or brightness acquisition unit
- 74:: color and/or brightness adjustment unit
- 76:: display pattern setting unit
- 80:: color and/or brightness storage unit
- 84:: composite image generation unit
- 100:: endoscope system
- 104:: blue laser light source
- 106:: blue-violet laser light source
- 108:: light source control unit
- 110:: fluorescent body
- 200:: endoscope system
- 202:: broadband light source
- 204:: rotation filter
- 206:: filter switching unit
- 208:: imaging sensor
- 210:: normal mode filter
- 210a:: Bb filter
- 210b:: G filter
- 210c:: R filter
- 212:: special mode filter
- 212a:: Bn filter
- 212b:: Gn filter
- 214:: mixed mode filter
- 214a:: Bb filter
- 214b:: G filter
- 214c:: R filter
- 214d:: Bn filter
- 214e:: Gn filter
- 300:: capsule endoscope
- 302:: light source
- 303:: light source control unit
- 304:: imaging sensor
- 305:: image signal processing unit
- 306:: image signal processing unit
- 308:: transmission and reception antenna

## Claims

1. An endoscope system comprising:
a light source that emits first mode illumination light and second mode illumination light;
an image acquisition unit that acquires a first mode display image and a second mode display image, the first mode display image being obtained by imaging an observation target including a first structure in a case where the observation target is illuminated with the first mode illumination light, and the second mode display image being obtained by imaging the observation target in a case where the observation target is illuminated with the second mode illumination light; and
an image correction unit that obtains a corrected image by correcting the first mode display image or the second mode display image, and performs correction such that a difference in a color and/or brightness of the first structure between the first mode display image and the second mode display image before the correction becomes small.

2. The endoscope system according to claim 1,
wherein the image correction unit includes
a color and/or brightness acquisition unit that acquires a color and/or brightness of the first structure from the first mode display image, and
a color and/or brightness adjustment unit that performs correction for adjusting a color and/or brightness of the second mode display image such that a difference between the color and/or the brightness of the first structure in the second mode display image before the correction and the color and/or the brightness of the first structure acquired by the color and/or brightness acquisition unit becomes small.

3. The endoscope system according to claim 1,
wherein the image correction unit includes
a color and/or brightness storage unit that stores the color and/or the brightness of the first structure in advance, and
a color and/or brightness adjustment unit that performs correction for adjusting the color and/or the brightness of the second mode display image such that a difference between the color and/or the brightness of the first structure in the second mode display image before the correction and the color and/or the brightness of the first structure stored in the color and/or brightness storage unit becomes small.

4. The endoscope system according to any one of claims 1 to 3,
wherein the observation target includes a second structure, and
the image correction unit corrects the first mode display image or the second mode display image such that a difference in the color and/or the brightness of the first structure and the second structure between the first mode display image and the second mode display image before the correction becomes small.

5. The endoscope system according to claim 4,
wherein the image correction unit includes
a structure extraction unit that extracts the second structure from the second mode display image to generate a second structure-extracted second mode display image, or extracts the first structure by removing the second structure from the second mode display image to generate a first structure-extracted second mode display image, and
a color and/or brightness adjustment unit that performs correction for adjusting the color and/or the brightness of the first structure in the second mode display image by referring to the first structure-extracted second mode display image, or performs correction for adjusting the color and/or the brightness of the second structure in the second mode display image by referring to the second structure-extracted second mode display image.

6. The endoscope system according to claim 4 or 5,
wherein the first structure is a mucous membrane structure, and the second structure is a vascular structure.

7. The endoscope system according to any one of claims 1 to 6, further comprising:
a display unit that displays the corrected image and a non-corrected image which is not corrected by the image correction unit among the first mode display image or the second mode display image; and
a display control unit that controls the display unit.

8. The endoscope system according to claim 7,
wherein the display control unit controls the display unit such that the display unit alternately displays the non-corrected image and the corrected image.

9. The endoscope system according to claim 7 or 8,
wherein the display control unit has a display pattern setting unit that sets a display pattern including a display time of the non-corrected image and a display time of the corrected image on the display unit.

10. The endoscope system according to any one of claims 7 to 9, further comprising:
a composite image generation unit that combines the non-corrected image and the corrected image to generate a composite image,
wherein the display unit displays the non-corrected image, the composite image, and the corrected image.

11. The endoscope system according to any one of claims 1 to 10,
wherein the first mode illumination light is light in which a single color or a plurality of colors of light is emitted under a first mode light emission condition, and
the second mode illumination light is light in which a single color or a plurality of colors of light is emitted under a second mode light emission condition that is different from the first mode light emission condition.

12. The endoscope system according to claim 11,
wherein in a case where the first mode illumination light is light in which the plurality of colors of light is simultaneously emitted, the first mode display image is an image obtained by imaging the observation target that is simultaneously illuminated with the plurality of colors of light, and
in a case where the first mode illumination light is light in which the plurality of colors of light is sequentially emitted, the first mode display image is an image obtained by sequentially imaging the observation target that is sequentially illuminated with the respective colors of light.

13. The endoscope system according to claim 11 or 12,
wherein in a case where the second mode illumination light is light in which the plurality of colors of light is simultaneously emitted, the second mode display image is an image obtained by imaging the observation target that is simultaneously illuminated with the plurality of colors of light, and
in a case where the second mode illumination light is light in which the plurality of colors of light is sequentially emitted, the second mode display image is an image obtained by sequentially imaging the observation target that is sequentially illuminated with the respective colors of light.

14. An endoscope system comprising:
a light source that emits first mode illumination light and second mode illumination light;
an image acquisition unit that acquires a first mode display image and a second mode display image, the first mode display image being obtained by imaging an observation target including a first structure in a case where the observation target is illuminated with the first mode illumination light, and the second mode display image being obtained by imaging the observation target in a case where the observation target is illuminated with the second mode illumination light;
an image correction unit that obtains a corrected image by correcting the first mode display image or the second mode display image, and performs correction such that a difference in a color and/or brightness of the first structure between the first mode display image and the second mode display image before the correction becomes small; and
a display control unit that controls a display unit,
wherein the image correction unit includes
a color and/or brightness acquisition unit that acquires a color and/or brightness of the first structure from the first mode display image, and
a color and/or brightness adjustment unit that performs correction for adjusting a color and/or brightness of the second mode display image such that a difference between the color and/or the brightness of the first structure in the second mode display image before the correction and the color and/or the brightness of the first structure acquired by the color and/or brightness acquisition unit becomes small,
the display control unit controls the display unit such that the display unit alternately displays the first mode display image, and the second mode display image of which the color and/or brightness is adjusted by the color and/or brightness adjustment unit.

15. A method of operating an endoscope system, the method comprising:
a step of, by a light source, emitting first mode illumination light and second mode illumination light;
a step of, by an image acquisition unit, acquiring a first mode display image and a second mode display image, the first mode display image being obtained by imaging an observation target including a first structure in a case where the first mode illumination light is emitted, and the second mode display image being obtained by imaging the observation target in a case where the second mode illumination light is emitted; and
a step of, by an image correction unit, obtaining a corrected image by correcting the first mode display image or the second mode display image, and performing correction such that a difference in a color and/or brightness of the first structure between the first mode display image and the second mode display image before the correction becomes small.
